# EUROPÄISCHE PATENTSCHRIFT

(11) **EP 2 326 571 B1**
(45) Veröffentlichungstag und Bekanntmachung des Hinweises auf die Patenterteilung: **29.07.2015**
(21) Anmeldenummer: 09778275.9
(22) Anmeldetag: 02.09.2009
(51) Int. Cl.: B65D 75/58

(54) **KINDERSICHERE EINZELDOSISVERPACKUNG FÜR TRANSDERMALE THERAPEUTISCHE SYSTEME ODER FOLIENFÖRMIGE DARREICHUNGSFORMEN**
CHILDPROOF INDIVIDUAL DOSE PACKAGING FOR TRANSDERMAL THERAPEUTIC SYSTEMS OR FILM-LIKE FORMS OF ADMINISTRATION
EMBALLAGE DE DOSE INDIVIDUELLE AVEC SÉCURITÉ ENFANT POUR DES SYSTÈMES THÉRAPEUTIQUES TRANSDERMIQUES OU DES FORMES GALÉNIQUES PELLICULAIRES

(30) Priorität: 08.09.2008 US 191362 P
(43) Veröffentlichungstag der Anmeldung: 01.06.2011
(73) Patentinhaber: LTS LOHMANN Therapie-Systeme AG, 56626 Andernach (DE)
(72) Erfinder: KRUMME, Markus, 56567 Neuwied (DE)
(86) Internationale Anmeldenummer: PCT/EP2009/006349
(87) Internationale Veröffentlichungsnummer: WO 2010/025899

(56) Entgegenhaltungen:
- DE-A1-102004 047 445
- DE-U1- 20 320 948
- DE-U1-202004 003 781

## Beschreibung

Die vorliegende Erfindung betrifft Einzeldosisverpackungen für transdermale therapeutische Systeme oder filmförmige beziehungsweise folienförmige Darreichungsformen, die leicht zu öffnen, aber dennoch kindersicher sind, gemäß dem Oberbegriff des Anspruchs 1.

Die vorliegende Erfindung umfaßt auch ein Verfahren zur Herstellung der erfindungsgemäßen Einzeldosisverpackungen, das sich durch einen sparsamen Materialeinsatz auszeichnet, gemäß Anspruch 11.

Arzneimittel sollten als Einzeldosis abgeteilt verpackt sein, damit gewährleistet werden kann, daß zu einer gewünschten Zeit nur eine vorbestimmte Dosis des zu verabreichenden Arzneistoffs eingenommen wird und eine unbeabsichtigte Mehrfachdosierung vermieden werden kann. Zudem sollten Arzneimittel während der Lagerung nach ihrer Herstellung vor schädlichen Umwelteinflüssen geschützt werden, um die Qualität des Arzneimittels zu erhalten. Diesen Anforderungen genügen Behälter, die mehrere Arzneimitteleinheiten enthalten, nicht, denn durch das wiederholte Öffnen des Behälters für die Entnahme des jeweils zur Verabreichung vorgesehenen Arzneimittels können versehentlich zu viele Arzneimitteleinheiten entnommen werden. Zudem wird die gesamte Haltbarkeitsdauer des Inhalts dieser Behälter durch die mit der Öffnung des Behälters einhergehende mechanische und/oder chemische Belastung der Arzneimitteleinheiten, beispielsweise durch Feuchtigkeit oder Sauerstoff, verkürzt. Die Beeinträchtigung der Haltbarkeit ist um so stärker, je empfindlicher das Arzneimittel oder der Arzneistoff auf die mechanische und/oder chemische Belastung reagiert.

Ein weiterer wesentlicher Aspekt bei der Verpackung von Arzneimitteln ist die Sicherheit für den Schutz von Personen vor einer unbeabsichtigten Selbstmedikation. Insbesondere Kinder verstehen Verpackungen aufgrund ihrer natürlichen Neugier als Aufforderung zum Öffnen, so daß sie unabsichtlich für sie gefährliche Substanzen freilegen können. Daher sollten Arzneimittelverpackungen so ausgestaltet sein, daß sie nicht unbeabsichtigt geöffnet werden können, insbesondere nicht von Kindern.

Allerdings sollen Verpackungen, insbesondere auch Arzneimittelverpackungen, leicht zu öffnen sei, damit beispielsweise auch Senioren ohne Probleme an ihre Arzneimittel gelangen können. Eine kindersichere, jedoch seniorenfreundliche, d. h. eine leicht zu öffnende Verpackung bereitzustellen, stellt den Verpackungsingenieur vor Probleme, deren Lösungen einander auszuschließen scheinen.

Aus dem Stand der Technik sind Vorschläge für leicht zu öffnende, aber kindersichere Verpackungen bekannt.

Die Offenlegungsschrift EP 1 626 010 A1 offenbart eine flexible Verpackung aus einer flexiblen Folie, die um ein Produkt gewickelt ist und an der längsseitigen Überlappung sowie den einander gegenüberliegenden Enden versiegelt ist, so daß ein Hohlraum für das Produkt entsteht. Die flexible Folie ist im wesentlichen reißfest, allerdings weist jede Verpackung einen gut positionierten, lokal begrenzten Schwächungsbereich auf, der ein manuelles Einreißen der flexiblen Folie und Öffnen der Verpackung ermöglicht.

Die Offenlegungsschrift US 2006/0138016 A1 betrifft eine Sicherheitsverpackung, bei der eine Packstoffbahn mit mindestens einer Mulde versehen ist, die der Aufnahme des Packguts dient, und eine darüberliegende Siegelbahn, die am Muldenrand miteinander verschweißt sind, wobei die Siegelbahn eine Festigkert hat, die ein Durchdrücken des Packguts verhindert, wenn von außen Druck auf die Packstoffbahn ausgeübt wird. Jede einzelne Verpackung weist zwei parallele Schwächungslinien auf, über die eine Ecke der Verpackung derart nach innen gefaltet werden kann, daß die Siegelbahn mit der spitzen Ecke der Verpackung perforiert werden kann, um das Packgut entnehmen zu können.

Im Stand der Technik werden Lösungen für die einzelnen zuvor angesprochenen Probleme beschrieben. So wird in der Offenlegungsschrift DE 10 2004 047 445 A1 eine nicht wiederverschließbare Verpackung für gesundheitsgefährdende Erzeugnisse offenbart, die zwei übereinanderliegend angeordnete Packstoffelemente, einen ersten Flächenabschnitt, an dessen Rand oder Rändern die beiden Packstoffelemente lösbar miteinander verbunden sind, wobei zwischen den beiden Packstoffelementen mindestens ein allseitig umschlossener Hohlraum zur Aufnahme des Packguts gebildet wird, und einen zweiten, außerhalb des ersten Flächenabschnitts liegenden oder an diesen angrenzenden Flächenabschnitt aufweist, an dessen Rand oder Rändern die beiden Packstoffelemente lösbar miteinander verbunden sind. Zumindest eines der beiden Packstoffelemente ist mit mindestens einer Struktur versehen, die innerhalb des zweiten Flächenabschnitts verläuft und die das Einreißen des/der Packstoffelemente ermöglicht.

In der Offenlegungsschrift US 2006/0023976 A1 werden aufziehbare Beutel für eine oder mehrere Dosen eines Arzneimittels beschrieben, bei denen zwei Packstoffbahnen randständig miteinander versiegelt sind, und die im Bereich des Siegelrands mit einer das Einreißen ermöglichenden Oberflächenstruktur versehen ist, welche von seiner Falzlinie gekreuzt wird. Der Rand des Beutels muß entlang der Falzlinie geknickt werden, damit er an der Oberflächenstruktur eingerissen und geöffnet werden kann.

In der Offenlegungsschrift DE 10 2006 041 921 A1 wird eine Verpackung für wirkstoffhaltige Filme beschrieben, die eine Trägerschicht und eine mit dieser lösbar verbundene Deckschicht umfaßt und in einer paarweisen Anordnung zwei gegenüberliegende, durch einen Steg voneinander getrennte Flächenbereiche aufweist, innerhalb welcher die Deckschicht nicht mit der Trägerschicht verbunden ist, wodurch zwei voneinander getrennte, allseitig umschlossene Räume zur paarweisen Aufnahme der genannten Filme gebildet werden. Innerhalb des genannten Stegs ist ein weiterer Flächenbereich vorhanden, in welchem die Trägerschicht nicht mit der Deckschicht verbunden ist, wodurch ein allseitig umschlossener Hohlraum gebildet wird. Innerhalb des Stegs ist mindestens eine Perforationslinie vorhanden. Bei den aus DE 10 2004 047 445 A1, US 2006/0023976 A1 und DE 10 2006 041 921 A1 bekannten Folienverpackungen wird die Aufgabe der Bereitstellung einer kindersicheren Verpackung, die gleichzeitig Schutz des Packguts vor chemischer Beeinträchtigung bietet, durch die Verwendung eines aufziehbaren, durch Heißsiegelung gefertigten Beutels aus zwei Folien gelöst, die jeweils eine dünne Aluminiumschicht enthalten. Die Folienverpackungen weisen einen seitlich angebrachten Schnitt auf, der die Beutelseite selbst aber nicht schneidet. Dadurch müssen die Beutel in der Mitte des Schnittes über einen Winkel von 90° hinaus gefaltet werden, um eine Rißkerbe in der Seite des Beutelrandes zu erzeugen. Damit wird eine Öffnungshilfe zum Anfassen freigelegt, mit deren Hilfe die beiden Folien des Beutels voneinander abgezogen werden können.

Das Verpacken filmförmiger oder folienförmiger Arzneimittel/Darreichungsformen stellt eine besondere Herausforderung dar, da Filme und Folien empfindlich auf chemische (Feuchte, Sauerstoff) und mechanische Beanspruchungen reagieren. Um Verpackungen mit der erforderlichen Schutzwirkung herzustellen, müssen sogenannte "Hochbarriere-Folien" als Verpackungsmaterial eingesetzt werden, die mechanisch belastbar sind und allenfalls eine geringe Permeation von Gasen und Feuchtigkeit zulassen. Diese Folien haben den Nachteil, daß sie teuer sind. Zudem ist die Fläche von filmförmigen oder folienförmigen Arzneiformen, verglichen mit anderen Arzneiformen für orale Anwendungen (Tabletten, Dragees, Kapseln, Pastillen und dergleichen), recht groß. Die erforderlichen Verpackungen für film- oder folienförmige Darreichungsformen sind flächenbezogen größer als die Darreichungsform selbst, wobei zudem zu berücksichtigen ist, daß die Packstoff-Folie sowohl die Oberseite als auch die Unterseite der filmförmigen oder folienförmigen Darreichungsform bedecken muß. Daher muß flächenbezogen mehr als doppelt so viel Packstoff wie zu verpackendes Gut verwendet werden. Ein ungünstiges Verhältnis von Verpackungskosten zu Produktkosten ist bei einzeln verpackten film- oder folienförmigen Darreichungsformen kaum zu vermeiden, so daß die Kosten für das Endprodukt höher sind als gewünscht.

Das Verpacken einzelner film- oder folienförmiger Darreichungsformen erfüllt zwar die Anforderungen an den Schutz für das Arzneimittel, hat aber den Nachteil, daß es in der praktischen Umsetzung sehr teuer ist, weil es eines hohen Materialeinsatzes bedarf und die entsprechenden Verpackungen nur vergleichsweise langsam produziert werden können. Zwar können durch den in DE 10 2006 041 921 A1 offenbarten, gepaarten Ansatz die Herstellungskosten durch eine Reduktion der Maschinenlaufzeit und des Materialeinsatzes verringert werden, aber der Nachteil bei diesem Ansatz besteht darin, daß eine kindersichere Verpackung nur bei Verpacken gepaarter Filme (filmförmige Darreichungsformen) gegeben ist. Das Auftrennen der Kindersicherung zur Freilegung einer Darreichungsform läßt die andere Darreichungsform zwar noch chemisch dicht verpackt, allerdings ist die Kindersicherung nicht mehr gegeben. Insofern ist die Verwendung einer Verpackung gemäß DE 10 2006 041 921 A1 nur angezeigt, wenn das Intervall zwischen der Einnahme der ersten Einzeldosis und der Einnahme der zweiten Einzeldosis nicht allzu groß ist.

Der vorliegenden Erfindung lag die Aufgabe zugrunde, eine leicht zu öffnende, aber kindersichere Verpackung für Einzeldosen in Form von film- beziehungsweise folienförmigen Darreichungsformen sowie für transdermale therapeutische Systeme (TTS) bereitzustellen, die gegenüber den bekannten Verpackungen einfacher und sicherer in der Handhabung und gleichzeitig kostengünstiger und/oder materialsparender herzustellen sind.

Die Aufgabe wird durch eine nicht wiederverschließbare Verpackung in Form eines aufziehbaren Siegelrandbeutels gelöst, der eine asymmetrisch angeordnete Anfaß- und Aufziehhilfe aufweist und der unter Verwendung eines siegelfähigen, reißfesten Packstoffs hergestellt wird, der eine niedrige Weiterreißfestigkeit aufweist. Die Anfaßhilfe fungiert als Kindersicherung, das heißt als Sicherung vor unbeabsichtigtem Öffnen der Verpackung, und ist so gestaltet, daß die Verpackung materialsparend aneinandergereiht gefertigt werden kann.

Die Anfaß- und Aufreißhilfe der erfindungsgemäßen Siegelrandbeutel ist eine bei Paarung flächenkomplementäre Lasche. Das bedeutet, daß sich die Laschen von zwei deckungsgleichen Siegelrandbeuteln flächenkomplementär zu einem viereckigen Flächenbereich, das heißt zu einem rechteckigen oder quadratischen Flächenbereich, ergänzen und zusammenlegen lassen. Das läßt sich dadurch erreichen, daß die Lasche asymmetrisch und außermittig am Siegelrandbeutel angeordnet ist.

Bei dem Packstoff für die Herstellung der Siegelrandbeutel handelt es sich vorzugsweise um einen Packstoff, der geringe Permeationsraten für Gase und Feuchtigkeit aufweist.

Für die Übernahme der unterschiedlichen Funktionen, die der Packstoff zu erfüllen hat, eignen sich Packstoffe mit einem mehrschichtigen Aufbau besonders gut. Bei diesen Packstoffen, bei denen die einzelnen Lagen zu einem Verbund zusammengefügt sind, vorzugsweise in Form eines Laminats, übernehmen die einzelnen Lagen des Packstoffs eine oder mehrere Funktionen. Für die erfindungsgemäßen Verpackungen können aber auch einlagige Filme oder Folien als Packstoff verwendet werden, sofern diese zumindest alle notwendigen Funktionen des Packstoffs übernehmen können.

Der Siegelrandbeutel besteht aus zwei übereinanderliegend angeordneten Packstoffelementen, einem ersten Packstoffelement und einem zweiten Packstoffelement, zwischen denen sich das Packgut befindet, beispielsweise das transdermale therapeutische System oder die filmbeziehungsweise folienförmige Darreichungsform. Das Packgut wird von einer umlaufenden und durchgängigen, also nicht unterbrochenen Siegelnaht zwischen den beiden Packstoffelementen eingeschlossen.

Der Packstoff für die Packstoffelemente der erfindungsgemäßen Siegelrandbeutel ist reißfest und weist gleichzeitig eine niedrige Weiterreißfestigkeit auf. Durch die Reißfestigkeit des Packstoffs ist es nicht möglich, die Verpackung manuell, das heißt ohne Hilfsmittel wie Schere, Messer oder Zähne, aufzureißen.

Allerdings kann ein bestehender Riß erweitert und an einer vorbestimmten, geschwächten Stelle, beispielsweise an einer Einkerbung, eine Rißfortpflanzung erreicht werden, so daß ein manuelles Weiterreißen ohne Hilfsmittel möglich ist.

Die Reißfestigkeit des Packstoffs beträgt mindestens 20 N, vorzugsweise mindestens 50 N und besonders bevorzugt mindestens 70 N. Vorzugsweise liegt die Reißfestigkeit des Packstoffs unter 2000 N, besonders bevorzugt unter 150 N und ganz besonders bevorzugt unter 100 N, gemessen an den zwei miteinander verbundenen Packstoffelementen, welche die Verpackung bilden.

Die Weiterreißfestigkeit des Packstoffs darf nicht zu gering sein, weil dann kein ausreichender Schutz des Packgutes mehr gewährleistet werden kann und die Gefahr besteht, daß die genannten Anfaßhilfen beim Öffnen der Verpackung abreißen. Dies läßt sich durch einfache Versuche ermitteln. Die Weiterreißfestigkeit des Packstoffs beträgt weniger als 10 N, vorzugsweise weniger als 2 N, besonders bevorzugt weniger als 1,5 N, und vorzugsweise über 0,5 N, gemessen an den zwei miteinander verbundenen Packstoffelementen, welche die Verpackung bilden.

Die Reißfestigkeit und die Weiterreißfestigkeit des Packstoffs können mittels bekannter Zugprüfmaschinen unter Verwendung einer Probenaufnahme für Einreißversuche (Typ No. 00740) ermittelt werden (z. B. erhältlich von FRANK Prüfgeräte GmbH, D-69488 Birkenau).

Um das Weiterreißen des Packstoffs zu ermöglichen oder zu erleichtern, beträgt die Reißfestigkeit ein Vielfaches der Weiterreißfestigkeit. Vorzugsweise liegt das Verhältnis von Reißfestigkeit zu Weiterreißfestigkeit im Bereich von 2 : 1 bis 200 : 1, besonders bevorzugt im Bereich von 50 : 1 bis 150 : 1, bezogen auf die Reißfestigkeit und Weiterreißfestigkeit der beiden miteinander verbundenen Packstoffelemente.

Packstoffe, welche die genannten Eigenschaften (Reißfestigkeit, Weiterreißfestigkeit) aufweisen, sind dem Fachmann bekannt, vorzugsweise handelt es sich hierbei um folienförmige, flexible Materialien aus Kunststoff, Metall (z. B. Aluminium), oder Verbundwerkstoffe aus den genannten Stoffen. Besonders geeignete Packstoffe sind Polyesterfolien. Als folienförmige Materialien können sowohl Monofolien als auch zwei- oder mehrschichtige Laminate verwendet werden. Als Kunststoffmaterialien kommen insbesondere folgende in Betracht, einzeln oder in Kombinationen: Polyester (z. B. Polyethylenterephthalat), Polyethylen (z. B. HDPE, LDPE), Polypropylen, Polyisobutylen, Polystyrol, Polyvinylchlorid, Polyamide, Polycarbonat, Cellulose-Acetat. Die Dicke der folienförmigen Materialien liegt vorzugsweise im Bereich von 5 bis 300 µm, besonders bevorzugt 50 bis 200 µm.

Ein weiteres, bevorzugtes Folienmaterial ist Barex^{®} (BP Chemicals), ein Copolymer aus Acrylnitril und Butadien. Aufgrund seiner guten Barriere-Eigenschaften und chemischen Widerstandsfähigkeit eignet es sich insbesondere zum Verpacken von Arzneimitteln mit einem Gehalt an aggressiven und/oder flüchtigen Wirkstoffen, z. B. Nicotin.

Der Packstoff für die erfindungsgemäße Verpackung muß siegelfähig sein, so daß die beiden Packstoffelemente miteinander verschweißt werden können, vorzugsweise durch Heißsiegelung, aber auch mit jedem anderen geeigneten Heißsiegelverfahren oder Kaltsiegelverfahren wie Ultraschallsiegeln, Lasersiegeln oder vergleichbare, dem Fachmann bekannte Folienschweißverfahren.

Geeignete siegelfähige Materialien müssen chemisch dicht gegen Feuchte und Luft sein, um den Schutz des Produktes vor äußeren Umweltfaktoren zu gewährleisten. Gleichzeitig müssen diese Materialien nach Siegelung eine Trennung des Verbundes der beiden Packstoffelemente zum Öffnen der Verpackung mit niedriger Aufreißkraft ermöglichen.

In einer bevorzugten Ausführungsform weist der Packstoff eine siegelfähige Schicht auf, die dem Produkt zugewandt ist. Derartige siegelfähige Schichten basieren auf Polyethylen und sind kommerziell erhältlich. Beispiele sind die von der Firma Amcor Flexibles vertriebenen Core-Peel^{™}-Technologie sowie vergleichbare Materialien auf Polyethylen-Basis, die von vielen Packmaterialherstellern, beispielsweise den Firmen Danapak, Huhtamaki, Alcan oder Klöckner, angeboten werden.

Die genannten lösbaren Verbindungen werden vorzugsweise durch Siegelnähte oder Siegelflächen gebildet, für die als Siegelmedium peelfähige Folienlacke (Peel-Lack) oder Schmelzkleber verwendet werden können. Geeignete Siegelmassen und Siegelverfahren sind dem Fachmann bekannt, z. B. Siegelmassen auf Basis von Polyethylen-LD oder Ethylen-Vinylacetat-Copolymeren.

Die Siegelnähte oder Siegelflächen weisen vorzugsweise eine Breite von 0,1 mm bis 10 cm auf, besonders bevorzugt ein Breite von 1 mm bis 2 cm und ganz besonders bevorzugt eine Breite von 2 mm bis 8 mm, und sie erstrecken sich vorzugsweise über die gesamte Länge oder Breite der Packstoffelemente. An besonders exponierten Stellen kann die Siegelnahtbreite auch größer sein. Um das Öffnen der Verpackung zusätzlich zu erschweren, kann mindestens eine der Siegelnähte breiter als die übrigen Siegelnähte ausgebildet sein.

Die Aufreißkraft für das Aufziehen gut trennbarer Siegelungen liegt im Bereich von 1-50 N/15 mm Siegelnahtbreite, vorzugsweise im Bereich von 3-20 N/15 mm. Das bedeutet, daß die Siegelnähte oder Siegelflächen vorzugsweise eine Festigkeit (= Siegelfestigkeit) im Bereich von 1 N/15 mm bis 50 N/15 mm, vorzugsweise 2 N/15 mm bis 20 N/15 mm, aufweisen.

In einer bevorzugten Ausführungsform ist der Packstoff für Gase und Feuchtigkeit nicht oder kaum permeabel, d. h. er weist geringe Permeationsraten für Gase und Feuchtigkeit auf. Vorzugsweise umfaßt der Packstoff eine Hochbarriereschicht in Form einer aluminiumhaltigen Folie. Der Packstoff kann aber auch jede andere Hochbarrierefolie aufweisen. Beispielhaft sind Folien aus Polychlorfluorethylen (PCTFE), kommerziell von der Firma Honeywell erhältlich unter dem Handelsnamen Aclar^{™}, Folien aus Cyclo-Olefin-Copolymeren (COC), die unter dem Markennamen Topas^{®} von Firma Ticona vertrieben werden, und Polyethylenterephthalat-Folien mit einer keramischen Beschichtung aus SiOₓ oder AlOₓ zu nennen.

Die beiden Packstoffelemente können aus denselben oder aus verschiedenartigen Materialien hergestellt sein. Vorzugsweise besteht mindestens eines der beiden Packstoffelemente aus transparentem Material (z. B. transparente Kunststoff-Folie).

Des weiteren umfaßt die Erfindung Ausführungsformen, bei denen ein Packstoffelement oder beide Packstoffelemente gleich oder unterschiedlich gefärbt sind, wobei es sich jeweils um eine transparente oder opake Färbung handeln kann. Beispielsweise kann eines der beiden Packstoffelemente aus einem nichttransparenten Verbundwerkstoff aus Papier (oder Pappe) mit Kunststoffen (z. B. mit Polyethylen oder Polyethylenterephthalat beschichtete Papiere), und das zweite Packstoffelement aus einer transparenten, farblosen oder gefärbten Kunststoff-Folie hergestellt sein. Zur Verminderung der Luft-, Licht- und Wasserdampfdurchlässigkeit ist es vorteilhaft, wenn zumindest eine Oberfläche der Trägerschicht oder/und der Deckschicht metallisiert ist (z. B. mit Aluminium beschichtet).

In einer besonderen Ausführungsform handelt es sich bei mindestens einem der Packstoffelemente des Siegelrandbeutels um einem Packstoff, der ein orientiertes Material enthält, also ein monoaxial gestrecktes Material. Bei Verwendung eines monoaxial gestreckten Materials ist die niedrige Reißfestigkeit quer zur Anfaßlasche ausgerichtet. Dadurch kann die Abtrennung der Kindersicherung erleichtert werden, wenn die Rißrichtung zum Abtrennen der Kindersicherung in Richtung der schwächeren Weiterreißfestigkeit liegt.

Ein Beispiel für einen besonders bevorzugten Packstoff ist ein Verbund aus monoaxial gestrecktem Polypropylen (50 µm - 70 µm dick), Aluminium (9 µm - 20 µm dick) und Polyethylen (20 µm - 100 µm dick).

Die Besonderheit der erfindungsgemäßen Siegelrandbeutel besteht in der Ausgestaltung einer als Anfaßhilfe dienenden Lasche an jedem der beiden Packstoffelemente, die das separate Anfassen der beiden Packstoffelemente ermöglicht und ohne die ein Öffnen der Verpackung nicht gelingen kann, da die Reißfestigkeit des Packstoffs dies verhindert.

Bei der Anfaßhilfe handelt es sich um eine flächenkomplementäre Lasche. Das bedeutet, daß sich die Laschen von zwei deckungsgleichen Siegelrandbeuteln bei ihrer Paarung zu einem viereckigen Flächenbereich ergänzen und-zusammenlegen lassen.

Die erfindungsgemäße Verpackung wird nachfolgend mit Bezug auf die Figuren erläutert. Dabei dienen die Figuren lediglich der Veranschaulichung der Erfindung, ohne sie jedoch auf das Dargestellte zu beschränken.

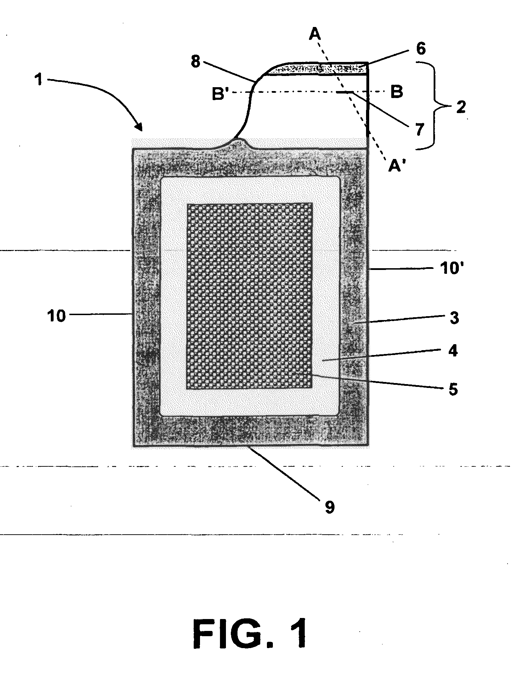

Figur 1 zeigt eine bevorzugte Ausführungsform der erfindungsgemäßen Einzeldosisverpackung in Draufsicht.
Figur 2A zeigt die Vorderkante einer bevorzugten Ausführungsform der erfindungsgemäßen Einzeldosisverpackung vor dem Öffnen.
Figur 2B stellt den Bereich der Einzeldosisverpackung gemäß Figur 2A dar, bei der die rechte obere Ecke der Lasche entlang der Linie A-A' in Figur 2A umgefaltet wurde, so daß die Struktur zum Einreißen des Packstoffs sein Weiterreißen entlang der Linie B-B' in Figur 2A ermöglicht.
Figur 2C stellt den Bereich der Einzeldosisverpackung gemäß Figur 2A dar, bei dem die Lasche an der Struktur, die ein Einreißen des Packstoffs ermöglicht, entlang der Linie B-B' in Figur 2A eingerissen wurde.
Figur 2D stellt den Bereich der Einzeldosisverpackung gemäß Figur 2A dar, nachdem die Kindersicherung abgerissen wurde, so daß die beiden als Anfaßhilfe dienenden Laschen der Packstoffelemente freiliegen.
Figur 3A veranschaulicht die Trennkräfte, die für das Öffnen einer aus dem Stand der Technik bekannten Verpackung erforderlich sind, die mit einer sich über die gesamte Breite des Siegelrandbeutels verlaufenden Anfaßhilfe versehen ist.
FIG. 3B veranschaulicht die Trennkräfte, die für das Öffnen einer aus dem Stand der Technik bekannten Verpackung erforderlich sind, bei der die zur Anfaßhilfe hin liegende Siegelnaht V-förmig ausgestaltet ist.
Figur 3C veranschaulicht die Trennkräfte, die für das Öffnen einer erfindungsgemäßen Verpackung erforderlich sind, die eine asymmetrische, über die halbe Breite des Siegelrandbeutels verlaufende Anfaßhilfe aufweist.
Figur 4 ist eine Skizze der Anordnung von Einzeldosisverpackungen gemäß der Erfindung bei ihrer Reihenfertigung aus Bandware.

Die Figuren 5A bis 5D veranschaulichen verschiedene Ausführungsformen von flächenkomplementären Laschen.

Figur 6 zeigt ein Verpackungspärchen, bei dem das punktsymmetrische Prinzip bei der paarweisen Anordnung von zwei Einzeldosisverpackungen verdeutlicht, wird.

Bei der erfindungsgemäßen Verpackung (1) handelt es sich um einen Siegelrandbeutel aus zwei übereinanderliegend angeordneten Packstoffelementen (11, 12), von denen ein Packstoffelement die Deckschicht und das andere Packstoffelement die Unterlage bildet, zwischen denen das Packgut (5), vorzugsweise ein transdermales therapeutisches System oder eine film- beziehungsweise folienförmige Darreichungsform, angeordnet ist. Die beiden Packstoffelemente sind derart miteinander versiegelt, daß das Packgut (5) von einem umlaufenden, durchgehenden Siegelrand (3) umgeben ist. Dadurch entsteht ein allseitig geschlossenes Kompartiment (4), in welchem das Packgut enthalten ist.

Der Siegelrandbeutel (1) weist eine Vorderkante (8), eine Hinterkante (9) und zwei vorzugsweise parallel verlaufende Seitenkanten (10, 10') auf.

Jedes der beiden übereinanderliegend angeordneten Packstoffelemente (11, 12), die über den Siegelrand (3) miteinander verbunden sind, weist eine Lasche (2) auf, die asymmetrisch randständig an der nachfolgend als Vorderende bezeichneten Seite des Siegelrandbeutels über den dortigen Siegelrand (3) hinausragt.

Die Laschen (2) der beiden Packstoffelemente sind im wesentlichen halb so breit wie der Siegelrandbeutel. Die Vorderkante (8) der erfindungsgemäßen Einzeldosisverpackung, die auf ihrer einen Hälfte vom dem vorderen Sigelrand (3) und-auf ihrer anderen Hälfte von den Laschen (2) gebildet wird, kann S-förmig, sigmoid, winkelig, schräg oder diagonal verlaufen.

Die Laschen sind flächenkomplementär ausgebildet, so daß sich zwei Laschen von deckungsgleichen Siegelrandbeuteln durch eine Paarung zu einem viereckigen Flächenbereich ergänzen und zusammenlegen lassen.

Die übereinanderliegenden Laschen der beiden Packstoffelemente sind an ihrem Ende, das dem Siegelrand (3) abgewandt ist, in einem schmalen, über die gesamte Breite der Laschen in diesem Bereich verlaufenden Streifen miteinander versiegelt. Dieser Siegelbereich (6) stellt einen wesentlichen Bestandteil der Kindersicherung der erfindungsgemäßen Verpackung dar.

Die Kindersicherung der Verpackung wird dadurch erreicht, daß die Laschen als erforderliche Anfaßhilfe zum Aufreißen der Verpackung erst durch Überwinden einer Kindersicherung freigelegt werden können. Diese Sicherung besteht darin, daß die beiden die Lasche bildenden Bereiche der Packstoffelemente durch ihre Versiegelung im Siegelbereich (6) nicht ohne weiteres voneinander separiert werden können. Aufgrund der Reißfestigkeit des Packstoffs ist ein manuelles Abreißen der Kindersicherung, das heißt ein Entfernen des Siegelbereichs (6), nicht möglich.

Das Freilegen der Anfaßhilfen erfolgt durch Abreißen der hinteren Versiegelung (6) nach Faltung der Laschen entlang der Linie A-A', die durch eine Struktur (7) zum Einreißen des Packstoffs in zumindest einem der beiden Packstoffelemente in dem Bereich der Laschen, in dem die beiden Packstoffelemente nicht miteinander versiegelt sind, führt. Hier kommt die niedrige Weiterreißfestigkeit zum Tragen, die erst das Abreißen der Verschweißung (6) ermöglicht.

Die Struktur (7) zum Einreißen des Packstoffs ist in dem Bereich der Lasche angeordnet, in dem die beiden Laschen nicht miteinander versiegelt sind. Die Struktur zum Einreißen des Packstoffs hat keinen Kontakt zum Rand beziehungsweise keine Verbindung zur Kante des Packstoffelements.

Die Struktur zum Einreißen des Packstoffs ermöglicht die Überwindung der initialen Reißfestigkeit, wenn die Struktur durch Falten der Lasche entlang der Linie A-A' Randberührung mit dem Umriß der Verpackung erlangt. Dann erst kann der Packstoff aufgrund seiner relativ niedrigen Weiterreißfestigkeit werkzeuglos entlang der Linien B-B' gerissen werden, um den Siegelbereich (6), der als Kindersicherung dient, abzureißen.

Die Struktur zum Einreißen des Packstoffs kann durch Einschnitt gerichtet ausgebildet sein, aber auch durch lokales Schwächen, z. B. durch Einwirkung von Strahlung wie beispielsweise in der EP 1 626 010 A1 beschrieben. Die Struktur zum Einreißen des Packstoffs erfolgt hierbei in dem Bereich der Lasche, in dem die beiden Packstoffelemente nicht miteinander verschweißt sind, vorzugsweise näher am Siegel bereich (6) als am Siegelrand (3), so daß nach Abreißen des Siegelbereichs (6) noch eine ausreichende Größe der Anfaßhilfen gegeben ist.

Durch diese Anordnung der Struktur zum Einreißen des Packstoff, weder im Bereich des Siegelrands noch innerhalb des Siegelrandbeutels, wird die Dichtigkeit der Verpackung nicht beeinträchtigt.

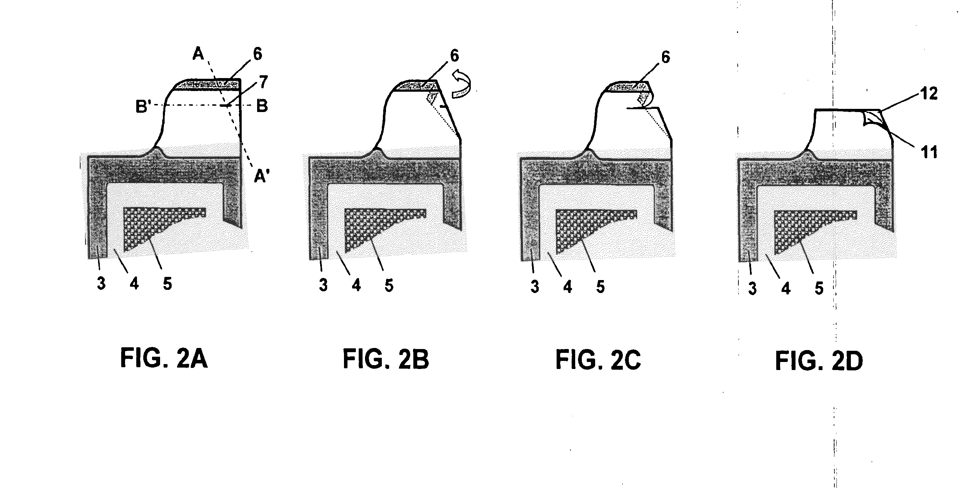

Die Struktur zum Einreißen des Packstoffs sollte erfindungsgemäß keine Randberührung mit der Verpackung haben, so daß diese Struktur erst durch Faltung der Lasche entlang einer durch diese Struktur verlaufenden Linie, beispielsweise entlang der Linie A-A' (FIG. 2A), den Rand des durch die Faltung entstehenden Umrisses schneidet. (FIG. 2B).

Als Strukturen, welche das Einreißen des/der Packstoffelemente ermöglichen, eignen sich vorzugsweise: gerade Schnitte, gezackte oder wellenförmige Schnitte, Perforationen, insbesondere Perforationen aus hintereinander angeordneten Punkten oder/und Schnitten, Materialaussparungen, Stanzungen, insbesondere pfeilförmige, dreieckige oder rautenförmige Stanzungen sowie Sollbruchstellen.

Die genannte Struktur, die das Einreißen des/der Packstoffelemente ermöglicht, kann in einem der beiden Packstoffelemente vorhanden sein oder in beiden, wobei die letztgenannte Ausführungsform bevorzugt wird. In diesem Fall sind die Strukturen zum Einreißen des Packstoffs in beiden Packstoffelementen vorzugsweise gleich oder ähnlich ausgestaltet und zueinander deckungsgleich angeordnet.

Bestimmungsgemäßer Gebrauch läßt durch die hohe Reißfestigkeit und Vermeidung von nach innen gerichteten Kerben im Umfang kein initiales Einreißen zu. Durch Faltung in der geschwächten Stelle der Längachse kann ein Einreißen erreicht und die verschweißte Stelle der Anfaßhilfe abgetrennt werden. Dadurch wird die Anfaßhilfe für beide Packstoffelemente greifbar und das Aufreißen kann über eine Ecke erfolgen. Durch das Aufreißen über eine Ecke werden sehr hohe Peelkraftmaxima im Vergleich zum Durchschnitt vermieden.

Das Öffnen der Verpackung erfolgt allgemein in der Weise, das die beiden Packstoffelemente entlang einer Linie, die durch die Struktur zum Einreißen des Packstoffs läuft, umgeknickt werden (FIG. 2A,), so daß die Struktur den Rand des sich durch das Umknicken ergebenden Umrisses der Verpackung berührt (FIG. 2B). Aufgrund der niedrigen Weiterreißfestigkeit kann dann die Kindersicherung (6) zunächst eingerissen (FIG. 2C) und nachfolgend abgerissen werden. Dadurch werden die Laschen der beiden Packstoffelemente separat zugänglich (FIG. 2D) und dienen nunmehr als Anfaß-Hilfen, um den Siegelrand zwischen den beiden Packstoffelementen aufzuziehen, wodurch das Packgut zugänglich wird.

Durch die erfindungsgemäße Kombination von Packstoff und Ausgestaltung der Kindersicherung ist es möglich, die Verpackung so zu gestalten, daß das Öffnen nur durch eine geordnete Abfolge von mindestens vier Schritten möglich ist:
(i) Falten oder Umknicken der Verpackung entlang einer Linie, wodurch die Schwächungsstruktur zum Einreißen zugänglich wird;
(ii) Einreißen der Verpackung an der nunmehr randständigen Schwächungsstruktur und Weiterreißen entlang dieser Struktur,
(iii) Ergreifen der freigewordenen Laschen der Packstoffelemente als Anfaßhilfen (Aufreiß-Laschen), und
(iv) Auseinanderziehen und Lösen des Siegelrands zwischen den Packstoffelementen.

Diese Handhabung ist für Kinder, insbesondere Kleinkinder, mit erheblichen Schwierigkeiten verbunden, jedoch für Erwachsene problemlos und ohne Zuhilfenahme von Werkzeugen möglich. In einer besonders bevorzugten Ausführungsform ist die Einzeldosisverpackung kindersicher gemäß DIN EN 14375 und/oder nach ASTM D3475-03a.

Die einzige Möglichkeit der werkzeuglosen Öffnung der Verpackung besteht darin, die Versiegelung der beiden Packstoffelemente aufzuziehen. Dafür ist die Existenz und ausreichende Größe der Anfaßhilfe notwendige Bedingung.

Die Laschen sollten mindestens 5 mm, vorzugsweise mindestens 10 mm und besonders bevorzugt mindestens 15 mm lang sein. Vorzugsweise sind die Laschen nicht länger als 30 mm und besonders bevorzugt nicht länger als 25 mm.

Die Breite der Laschen beträgt im wesentlichen das 0,5-fache der Verpackungsgesamtbreite, vorzugsweise mindestens 5 mm. In besonders bevorzugten Ausführungsformen beträgt die Breite der Lasche mindestens 10 mm und ganz besonders bevorzugt mindestens 15 mm. In besonders bevorzugten Ausführungsformen sind die Laschen nicht breiter als 50 mm und in ganz besonders bevorzugten Ausführungsformen nicht breiter als 30 mm.

In einer ebenfalls möglichen Ausführung unter Verwendung eines Hochbarrierepackstoffs (z.B. aluminiumhaltig) besteht eine erfindungsgemäße Besonderheit darin, daß eine Markierung der Verpackung, z.B. durch eine Chargennummer, durch Lasermarkierung oder Prägen im Bereich der Anfaßlache aufgebracht sein kann. Der besondere technische Nutzen besteht darin, daß durch die Lasermarkierung oder Prägung die Dichtigkeit der Verpackung im Beutel selber nicht gefährdet werden kann, selbst dann nicht, wenn beim Lasern durch Produkttoleranzen die Barriereschicht durchschossen wird oder durch Prägung die Aluminiumbarriere zerstört wird.

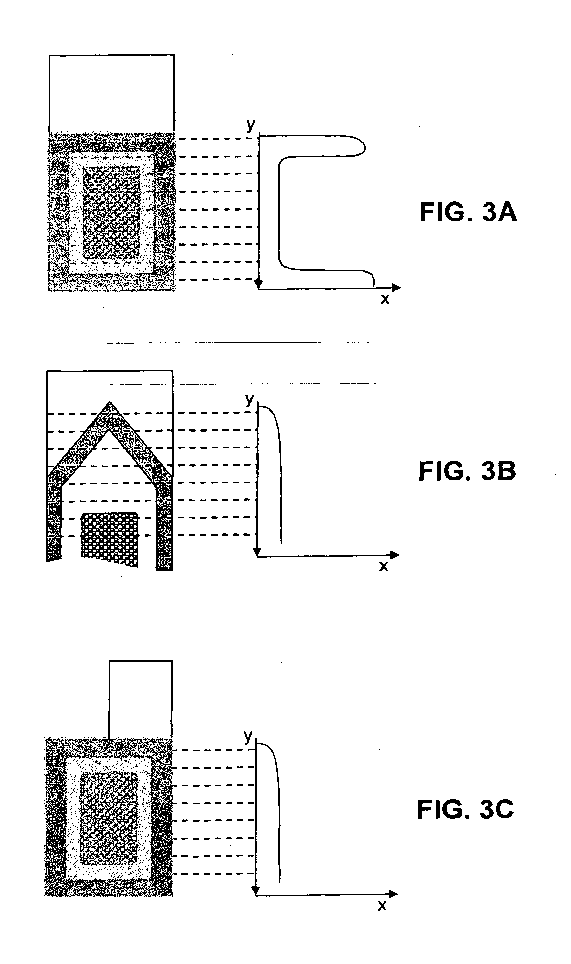

Die außermittige Anordnung der Lasche erzwingt den Beginn des Aufziehprozesses über eine der Ecken (FIG. 3C). Dadurch ist während des gesamten Fortschritts (y) des Aufziehvorgangs die zum Aufziehen des Siegelrandbeutels erforderliche Trennkraft (X) kleiner als bei einer mittigen Anordnung der Anfaßhilfen und dadurch bedingtem Aufziehen der Siegelung unter 90°, wie bei konventionellen Verpackungen gemäß FIG. 3A.

Beim Aufziehen einer bekannten Verpackung, wie sie in FIG. 3A dargestellt ist, muß zunächst die Siegelnaht an der Vorderseite über ihre ganze Breite aufgezogen werden, Dafür ist eine erheblich größere Trennkraft erforderlich als für das Aufziehen der Siegelnaht im Bereich der Seiten. (FIG. 3A).

Im Unterschied dazu erlaubt die erfindungsgemäße Anordnung der Anfaßhilfen ein zumindest initial leichteres und dadurch gleichmäßigeres Aufziehen des Siegelrandbeutels. Aus dem Stand der Technik ist bekannt, die für ein Aufziehen von Siegelrandbeuteln erforderliche Trennkraft durch Bereitstellen einer V-förmigen Siegelnaht zu verringern (FIG. 3B). Gegenüber diesem Stand der Technik wird durch die erfindungsgemäße Ausgestaltung (FIG. 3C) eine erhebliche Reduktion des notwendigen Packstoffs bei gleicher Funktionalität erreicht.

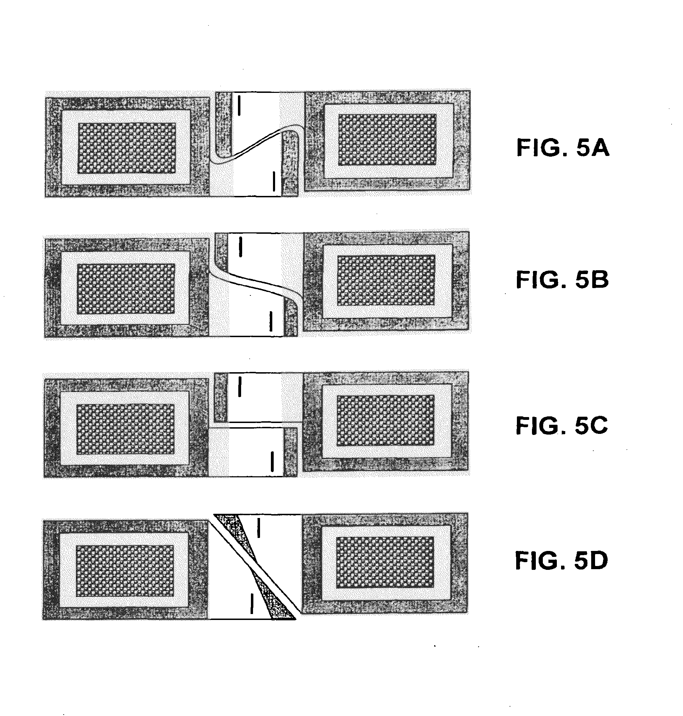

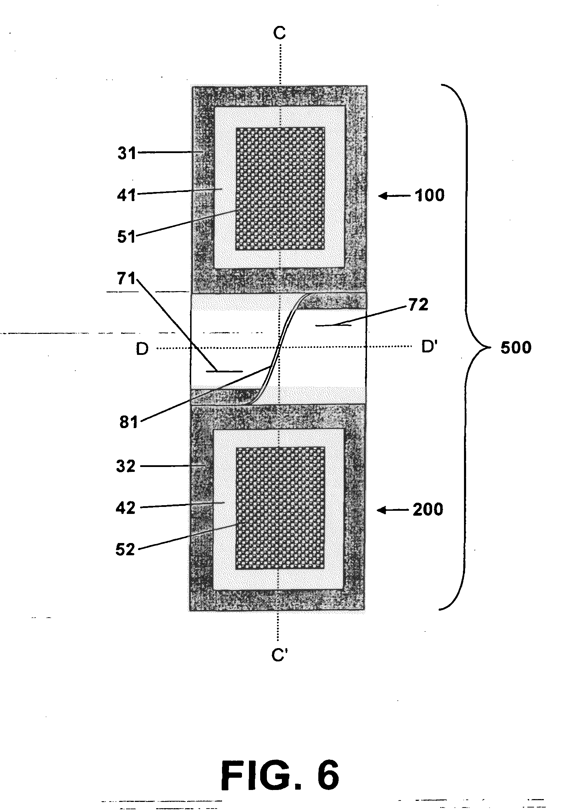

Die Anfaß- und Aufreißhilfe der erfindungsgemäßen Siegelrandbeutel ist eine bei Paarung flächenkomplementäre Lasche. Das bedeutet, daß sich die Laschen von zwei deckungsgleichen Siegelrandbeuteln flächenkomplementär zu einem viereckigen Flächenbereich ergänzen und zusammenlegen lassen. (FIG. 5A bis 5D, FIG. 6). Das läßt sich dadurch erreichen, daß die Lasche asymmetrisch und außermittig am Siegelrandbeutel angeordnet ist.

Die Figuren 5A bis 5D zeigen unterschiedliche Ausführungsformen der flächenkomplementären Laschen, die beispielsweise durch einen S-förmigen oder sigmoiden (FIG. 5A und 5B), winkeligen (FIG. 5C), schrägen oder diagonalen (FIG. 5D) Schnitt in dem Flächenabschnitt erzeugt werden können, in dem die Packstoffbahnen nicht miteinander versiegelt sind.

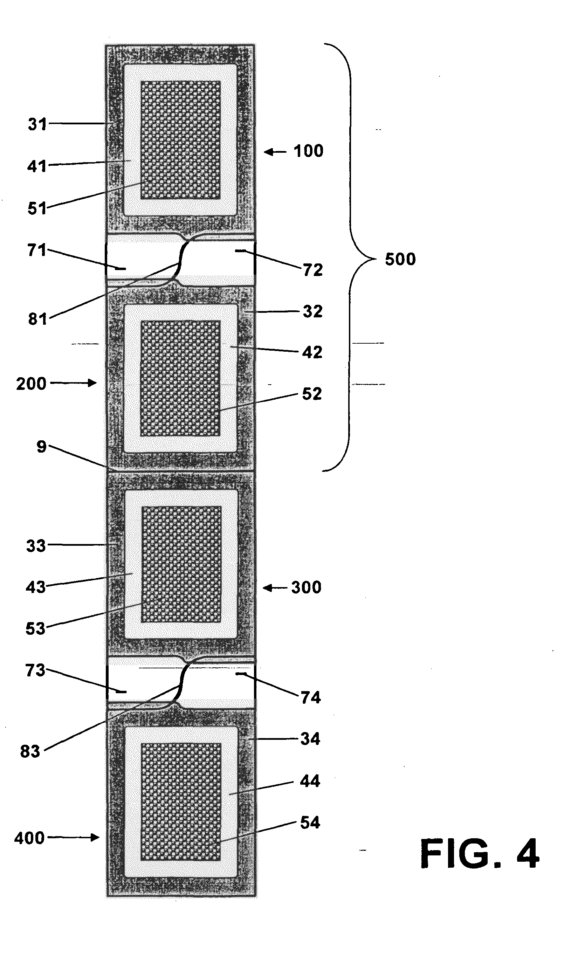

Die vorliegende Erfindung betrifft auch ein Verfahren zum Herstellen einer Einzeldosisverpackung für transdermale therapeutische Systeme oder filmförmige Darreichungsformen. Dieses Verfahren zeichnet sich dadurch aus, daß es im Vergleich zu den bekannten Verfahren besonders materialsparend ist. Durch die flächenkomplementäre Ausgestaltung der Laschen lassen sich die Einzeldosisverpackungen aus bahnförmigem Packstoff fertigen, ohne daß Packstoff als Abfall anfällt. Die Herstellung der erfindungsgemäßen Einzeldosisverpackungen kann somit ohne Verlust von Packstoffmaterial erfolgen. FIG. 4 veranschaulicht eine abfallfreie Fertigung der Einzeldosisverpackungen aus Packstoffbahnen, bei der eine Vielzahl von Verpackungspärchen (500) aus jeweils zwei Einzeldosisverpackungen, die im Bereich ihrer Laschen zusammenhängen, aufeinander folgen.

Die beiden Einzeldosisverpackungen eines Verpackungspärchen hängen hierbei punktsymmetrisch mit ihren Laschen zusammen. FIG. 6 veranschaulicht diese punktsymmetrische Anordnung von zwei Einzeldosisverpackungen durch die gepunkteten Linien C-C' und D-D', deren Schnittpunkt das Symmetriezentrum angibt.

Das erfindungsgemäße Verfahren umfaßt die folgenden Schritte:
- Bereitstellen einer ersten Packstoffbahn;
- Bereitstellen einer zweiten Packstoffbahn;
- Positionieren des Packgutes auf einer der beiden Packstoffbahnen;
- Übereinanderlegen und Verbinden der beiden Packstoffbahnen in der Weise, daß für jedes Packgut ein allseitig geschlossenes Kompartiment zur Aufnahme des Packgutes gebildet wird, an dessen Rand oder Rändern die beiden Packstoffelemente lösbar miteinander verbunden werden, wobei jeweils zwei in Bandrichtung aufeinanderfolgende Einzeldosisverpackungen derart paarweise angeordnet sind, daß ihre einander zugewandten Siegelränder unmittelbar aneinandergrenzen, und wobei zwischen den zwei aufeinanderfolgenden Einzeldosisverpackungen eines Pärchens ein Flächenabschnitt verbleibt, an dem die beiden Packstoffbahnen nicht miteinander versiegelt sind;
- Anbringen von zwei Strukturen zum Einreißen des Packstoffs in mindestens eine der beiden Packstoffbahnen, wobei diese Strukturen an den gegenüberliegenden Längskanten innerhalb des Flächenabschnitts angebracht werden, an dem die beiden Packstoffbahnen nicht miteinander versiegelt sind; und
- Vereinzeln der der aufeinander folgenden Verpackungs-Pärchen durch einen Schnitt entlang einer Linie, die quer zur Bahnrichtung der Packstoffbahnen verläuft, und Vereinzeln der Einzeldosisverpackungen eines Verpackungspärchens, indem die zwei Einzeldosisverpackungen eines Verpackungspärchens durch einen Schnitt entlang einer Linie abgeteilt werden, die auf der einen Längshälfte der miteinander verbundenen Packstoffbahnen im Bereich des vorderen Siegelrands einer Einzeldosisverpackung eines Verpackungspärchens und auf der anderen Längshälfte der miteinander verbundenen Packstoffbahnen im Bereich des vorderen Siegelrand der anderen Einzeldosisverpackung des Verpackungspärchens verläuft, so daß flächenkomplementäre Laschen entstehen.

Die vorstehend angegebene Reihenfolge der Verfahrensschritte ist nicht zwingend; beispielsweise können die Strukturen zum Einreißen des Packstoffs schon nach dem ersten oder zweiten oben angegebenen Schritt angebracht werden. Der Begriff "Schnitt" umfaßt alle dem Fachmann bekannten Verfahren zum Schneiden von Kunststofffolien, einschließlich Stanzen.

Vorzugsweise wird die aufziehbare Verbindung zwischen den Packstoffelementen durch Heißsiegeln bei Temperaturen im Bereich zwischen 50 °C und 200 °C, insbesondere 50 °C bis 90 °C, unter Verwendung von "Hotmelts" erzeugt. Die aufziehbare Verbindung zwischen den beiden Packstoffbahnen kann aber auch durch andere Heißsiegel- oder Kaltsiegelverfahren wie Ultraschallsiegeln, Lasersiegeln oder dergleichen erzeugt werden.

Die erfindungsgemäßen Verpackungen lassen sich durch geeignete komplementäre Geometrie zweier benachbarter Einzeldosisverpackungen (im Sinne von Escher-Figuren) materialsparend herstellen. Bei der erfindungsgemäßen Ausgestaltung der Laschen, die im wesentlichen halb so breit wie die Einzeldosisverpackung sind, lassen sich die Laschen von zwei aufeinanderfolgenden Einzeldosisverpackungen aus dem selben Abschnitt der Packstoffbahn fertigen, wenn die eine Packung um 180° um ihre Hochachse, das heißt um die Achse, die quer zur Flächenebene der Einzeldosisverpackung beziehungsweise der Packstoffbahnen verläuft, gedreht angeordnet ist. Die beiden derart um ein Symmetriezentrum angeordneten Einzeldosisverpackungen bilden ein Verpackungspärchen, von dem die mit ihren Vorderkanten aneinandergrenzenden Einzeldosisverpackungen durch einen einzigen Schnitt verlustlos in zwei identische Einzelpackungen getrennt werden können, die flächen- und formmäßig identisch sind (FIG. 4). Damit wird eine effektive Nutzung des Packstoffes erreicht, da die Anfaßhilfe nicht über die gesamte Breite der Verpackung reichen muß, sondern je zwei in Maschinenrichtung benachbarte Verpackungen sich eine erforderliche Vorzugslänge für die Anfaßhilfe teilen. Dadurch wird der Materialverbrauch für die Anfaßhilfe halbiert. Die Einzeldosisverpackungen sind aber durch die komplementäre Gestaltung identisch. Die mögliche Länge der Anfaßhilfe, und damit die Möglichkeit der Kraftausübung beim Aufreißen der Beutel, kann bei gleichem Materialverbrauch doppelt so groß sein, wie die Länge einer Anfaßhilfe bei Einzeldosisverpackungen, die ohne die erfindungsgemäße komplementäre gepaarte Anordnung hergestellt werden.

Die Verpackung kann aus Bandware durch Reihenfertigung auf rotativen Siegelmaschinen effizient gefertigt werden. Eine Siegelung, die elegant durch einen Werkzeugsatz erreicht werden kann, siegelt ein Siegelrandbeutel-Pärchen (500), wobei die beiden das Pärchen formenden Einzelverpackungen (100, 200) im Bereich der Anfaßhilfe zusammenhängen und im weiteren Verlauf des Herstellungsverfahrens voneinander getrennt werden. Die erfindungsgemäße Anfaßhilfe kann durch einen Schnitt entlang einer S-förmige, sigmoid, winkelig, schräg oder diagonal durch den Flächenabschnitt verlaufenden Linie, in dem die beiden Packstoffbahnen nicht miteinander versiegelt sind, erzeugt werden. Somit erfolgt der Schnitt in dem Leerraum zwischen den beiden das Pärchen bildenden Einzeldosisverpackungen derart, daß die Verschweißung am distalen Ende der Anfaßhilfe der einen Einzeldosisverpackung des Pärchens im Siegelrand der anderen Einzeldosisverpackung des Pärchens untergebracht ist und umgekehrt. Dadurch ist die Toleranz der Schnittlage in relativ weiten Bereichen (bei dem hier vorliegenden Beispiel ca. 1-2 mm Toleranzlage) nicht kritisch für die Kindersicherung.

Bei der Reihenfertigung aus Bandware folgen mehrere der vorgenannten Pärchen aus zwei Einzeldosisverpackungen aufeinander, derart, daß ein Pärchen mit einem quer zur Bahnrichtung verlaufenden Schnitt entlang der Linie (9) von dem darauf folgenden Pärchen abgetrennt werden kann.

## Patentansprüche

1. Einzeldosisverpackung für transdermale therapeutische Systeme oder folienförmige Darreichungsformen in Form eines aufziehbaren Siegelrandbeutels, umfassend zwei Packstoffelemente, die übereinanderliegend angeordnet sind, von denen mindestens ein Packstoffelement aus einem reißfesten Packstoff besteht, der eine niedrige Weiterreißfestigkeit aufweist,
wobei die beiden Packstoffelemente (11, 12), die durch eine durchgehende, um das Packgut (5) herumführende Siegelnaht (3) miteinander zu einem Siegelrandbeutel (1) verbunden sind, an einem Ende des Siegelrandbeutels (1) eine Lasche (2) aufweisen, die über den Siegelrand (3) hinausragt,
wobei mindestens eines der Packstoffelemente (11, 12) eine Struktur (7) zum Einreißen des Packstoffs in dem Bereich der Laschen (2) aufweist, in dem die Laschen (2) nicht miteinander versiegelt sind, und wobei die Struktur (7) zum Einreißen des Packstoffs ohne Kontakt zur Kante der Verpackung ist,
**dadurch gekennzeichnet,**
**dass** die Laschen (2) im Wesentlichen halb so breit wie der Siegelrandbeutel (1) sind und an ihrem Ende, das vom Siegelrand (3) abgewandt ist, einen Siegelbereich (6) aufweisen, an dem sie miteinander verbunden sind.

2. Einzeldosisverpackung nach Anspruch 1,
**dadurch gekennzeichnet,**
**dass** sich die Laschen (2) von zwei deckungsgleichen Siegelrandbeuteln (1), flächenkomplementär zu einem viereckigen Flächenbereich ergänzen und zusammenlegen lassen.

3. Einzeldosisverpackung nach Anspruch 1 oder 2,
**dadurch gekennzeichnet,**
**dass** die Lasche (2) asymmetrisch und außermittig am Siegelrandbeutel (1) angeordnet ist.

4. Einzeldosisverpackung nach einem der vorhergehenden Ansprüche,
**dadurch gekennzeichnet,**
**dass** die Reißfestigkeit des Packstoffs zwischen 20 N und 2000 N liegt, vorzugsweise zwischen 50 N und 150 N und besonders bevorzugt zwischen 70N und 100 N, gemessen an den zwei miteinander verbundenen Packstoffelementen (11, 12), die die Einzeldosisverpackung bilden.

5. Einzeldosisverpackung nach einem der vorhergehenden Ansprüche,
**dadurch gekennzeichnet,**
**dass** die Weiterreißfestigkeit weniger als 10 N beträgt, vorzugsweise weniger als 2 N und besonders bevorzugt weniger als 1,5 N, gemessen an den zwei miteinander verbundenen Packstoffelementen (11, 12), die die Einzeldosisverpackung bilden.

6. Einzeldosisverpackung nach einem der vorhergehenden Ansprüche,
**dadurch gekennzeichnet,**
**dass** das Verhältnis von Reißfestigkeit zu Weiterreißfestigkeit im Bereich von 2 : 1 bis 200: 1, besonders bevorzugt im Bereich von 50:1 bis 150 : 1 liegt, bezogen auf die Reißfestigkeit und Weiterreißfestigkeit von zwei miteinander verbundenen Packstoffelementen (11, 12), die die Einzeldosisverpackung bilden.

7. Einzeldosisverpackung nach einem der vorhergehenden Ansprüche,
**dadurch gekennzeichnet,**
**dass** die Aufreißkraft für das Aufziehen der beiden miteinander verbundenen Packstoffelemente (11, 12) im Bereich von 1-50 N/15 mm Siegelnahtbreite liegt, vorzugsweise Bereich von 3-20 N/15 mm Siegelnahtbreite.

8. Verpackung nach einem der vorhergehenden Ansprüche,
**dadurch gekennzeichnet,**
**dass** die Struktur (7) zum Einreißen des Packstoffs aus der Gruppe von Strukturen ausgewählt ist, die aus geraden Schnitten, gezackten Schnitten, wellenförmigenSchnitten, Perforationen, insbesondere aus hintereinander angeordneten Löchern oder Schnitten. Materialaussparungen, Stanzungen, insbesondere pfeilförmige, dreieckige und rautenförmige Stanzungen, und Sollbruchstellen besteht.

9. Verpackung nach einem der vorhergehenden Ansprüche,
**dadurch gekennzeichnet,**
**dass** zumindest eines der Packstoffelemente (11, 12), vorzugsweise beide Packstoffelemente (11, 12), aus einer einlagigen oder einer mehrlagigen Folie gebildet ist/sind.

10. Verpackung nach einem der vorhergehenden Ansprüche,
**dadurch gekennzeichnet,**
**dass** das darin verpackte Packgut (5) Kindern ohne Werkzeug nicht zugänglich ist und insbesondere kindersicher gemäß DIN EN 14375 und/oder nach ASTM D3475-03a ist.

11. Verfahren zur Herstellung einer Einzeldosisverpackung gemäß einem der vorhergehenden Ansprüche, umfassend die Schritte:
- Bereitstellen einer ersten Packstoffbahn;
- Bereitstellen einer zweiten Packstoffbahn;
- Positionieren des Packgutes (5) auf einer der beiden Packstoffbahnen;
- Übereinanderlegen und Verbinden der beiden Packstoffbahnen in der Weise, dass für jedes Packgut (5) ein allseitig geschlossenes Kompartiment (4) zur Aufnahme des Packgutes (5) gebildet wird, an dessen Rand oder Rändern die beiden Packstoffelemente (11, 12) lösbar miteinander verbunden werden, wobei jeweils zwei in Bandrichtung aufeinanderfolgende Einzeldosisverpackungen derart paarweise angeordnet sind, dass ihre einander zugewandten Siegelränder unmittelbar aneinandergrenzen, und wobei zwischen den zwei aufeinanderfolgenden Einzeldosisverpackungen eines Pärchens ein Flächenabschnitt verbleibt, an dem die beiden Packstoffbahnen nicht miteinander versiegelt sind;
- Anbringen von zwei Strukturen (7) zum Einreißen des Packstoffs in mindestens eine der beiden Packstoffbahnen, wobei diese Strukturen (7) an den gegenüberliegenden Längskanten innerhalb des Flächenabschnitts angebracht werden, an dem die beiden Packstoffbahnen nicht miteinander versiegelt sind; und
- Vereinzeln der aufeinander folgenden Verpackungs-Pärchen durch einen Schnitt entlang einer Linie, die quer zur Bahnrichtung der Packstoffbahnen verläuft, und Vereinzeln der Einzeldosisverpackungen eines Verpackungspärchens, indem die zwei Einzeldosisverpackungen eines Verpackungspärchens durch einen Schnitt entlang einer Linie abgeteilt werden, die auf der einen Längshälfte der miteinander verbundenen Packstoffbahnen im Bereich des vorderen Siegelrands einer Einzeldosisverpackung eines Verpackungspärchens und auf der anderen Längshälfte der miteinander verbundenen Packstoffbahnen im Bereich des vorderen Siegelrands der anderen Einzeldosisverpackung des Verpackungspärchens verläuft, so dass flächenkomplementäre Laschen (2) entstehen.

12. Verfahren nach Anspruch 11,
**dadurch gekennzeichnet,**
**dass** der Schnitt zur Vereinzelung der beiden Verpackungspärchen bildenden Einzeldosisverpackungen S-förmig, sigmoid, winkelig, schräg oder diagonal durch den Flächenabschnitt verläuft, in dem die beiden Packstoffbahnen nicht miteinander versiegelt sind.

13. Verfahren nach Anspruch 11 oder 12,
**dadurch gekennzeichnet,**
**dass** die Packstoffbahnen mittels Siegelnähten oder Siegelflächen, insbesondere unter Verwendung eines Siegellacks, miteinander verbunden werden.

14. Verfahren nach einem der Ansprüche 11 bis 13,
**dadurch gekennzeichnet,**
**dass** beide Packstoffbahnen in dem Abschnitt, in dem die Packstoffbahnen nicht miteinander versiegelt sind, mit mindestens einer Struktur (7) zum Einreißen des Packstoffs versehen werden, wobei diese Struktur(en) (7) vorzugsweise gleich ausgestaltet und zueinander deckungsgleich angeordnet werden.

15. Verfahren nach einem der Ansprüche 11 bis 14,
**dadurch gekennzeichnet,**
**dass** die Struktur(en) (7) zum Einreißen des Packstoffs aus der Gruppe von Strukturen (7) ausgewählt ist, die aus geraden Schnitten, gezackten Schnitten, wellenförmigen Schnitten, Perforationen, insbesondere aus hintereinander angeordneten Löchern oder Schnitten, Materialaussparungen, Stanzungen, insbesondere pfeilförmige, dreieckige und rautenförmige Stanzungen und Sollbruchstellen besteht.

## Claims

1. Single-dose pack for transdermal therapeutic systems or sheet-like forms of administration in the form of a peel-open sealed-edge bag, comprising two packaging-material elements located one above the other, of which at least one packaging-material element consists of a tear-resistant packaging material with a low tear-propagation resistance,
wherein the two packaging-material elements (11, 12) are connected to one another to form a sealed-edge bag (1) by a continuous sealing seam (3) leading around the contents (5) and, at one end of the sealed-edge bag (1), they have a lug (2) which projects beyond the sealed edge (3), wherein at least one of the packaging-material elements (11, 12) has a structure (7) for tearing the packaging material in that region of the lugs (2) in which the lugs (2) are not sealed to one another, and wherein the structure (7) for tearing the packaging material is not in contact with the periphery of the pack,
**characterized**
**in that** the lugs (2) are essentially half the width of the sealed-edge bag (1) and, at their end which is directed away from the sealed edge (3), have a sealing region (6), at which they are connected to one another.

2. Single-dose pack according to Claim 1, **characterized**
**in that** the lugs (2) of two congruent sealed-edge bags (1) can supplement one another, and be combined, with complementary surface areas to form a quadrilateral surface-area region.

3. Single-dose pack according to Claim 1 or 2, **characterized**
**in that** the lug (2) is arranged asymmetrically and eccentrically on the sealed-edge bag (1).

4. Single-dose pack according to one of the preceding claims,
**characterized**
**in that** the tear resistance of the packaging material is between 20 N and 2000 N, preferably between 50 N and 150 N and particularly preferably between 70 N and 100 N, as measured on the two interconnected packaging-material elements (11, 12) forming the single-dose pack.

5. Single-dose pack according to one of the preceding claims,
**characterized**
**in that** the tear-propagation resistance is less than 10 N, preferably less than 2 N and particularly preferably less than 1.5 N, as measured on the two interconnected packaging-material elements (11, 12) forming the single-dose pack.

6. Single-dose pack according to one of the preceding claims,
**characterized**
**in that** the ratio of tear resistance to tear-propagation resistance ranges from 2:1 to 200:1, particularly preferably ranges from 50:1 to 150:1, as measured in relation to the tear resistance and tear-propagation resistance of two interconnected packaging-material elements (11, 12) forming the single-dose pack.

7. Single-dose pack according to one of the preceding claims,
**characterized**
**in that** the tear-open force for peeling open the two interconnected packaging-material elements (11, 12) ranges from 1-50 N/15 mm sealing-seam width, and preferably ranges from 3-20 N/15 mm sealing-seam width.

8. Pack according to one of the preceding claims,
**characterized**
**in that** the structure (7) for tearing the packaging material is selected from the group of structures which comprises straight cuts, serrated cuts, undulating cuts, perforations, in particular successive holes or cuts, material cutouts, punched formations, in particular arrow-shaped, triangular and diamond-shaped punched formations, and predetermined breaking points.

9. Pack according to one of the preceding claims,
**characterized**
**in that** at least one of the packaging-material elements (11, 12), preferably both packaging-material elements (11, 12), is/are formed from a single-layer or multilayer sheet material.

10. Pack according to one of the preceding claims,
**characterized**
**in that** the contents (5) packaged therein are not accessible to children without tools being used and, in particular, are child-resistant in accordance with DIN EN 14375 and/or in accordance with ASTM D3475-03a.

11. Method of producing a single-dose pack according to one of the preceding claims, comprising the following steps:
- providing a first packaging-material web;
- providing a second packaging-material web;
- positioning the contents (5) on one of the two packaging-material webs;
- placing the two packaging-material webs one above the other, and connecting them, so as to form, for each contents unit (5), a compartment (4) which is intended for accommodating the contents (5), is closed all the way round and at the edge or edges of which the two packaging-material elements (11, 12) are connected to one another in a releasable manner, wherein in each case two single-dose packs which follow one after the other in the band direction are arranged in pairs such that their facing sealing edges are directly adjacent to one another, and wherein the two successive single-dose packs of a pair have remaining between them a surface-area portion at which the two packaging-material webs are not sealed to one another;
- applying, in at least one of the two packaging-material webs, two structures (2) for tearing the packaging material, wherein these structures (7) are applied to the opposite longitudinal peripheries within the surface-area portion at which the two packaging-material webs are not sealed to one another; and
- separating the successive pairs of packs using a cut along a line which runs transversely to the direction of the packaging-material webs, and separating the single-dose packs of a pair of packs by virtue of the two single-dose packs of a pair of packs being divided using a cut along a line which, on the one longitudinal half of the interconnected packaging-material webs, runs in the region of the front sealed edge of one single-dose pack of a pair of packs and, on the other longitudinal half of the interconnected packaging-material webs, runs in the region of the front sealed edge of the other single-dose pack of the pair of packs, lugs (2) with complementary surface areas being produced as a result.

12. Method according to Claim 11,
**characterized**
**in that** the cut for separating the two single-dose packs, which form pairs of packs, runs in an S-shaped, sigmoidal, angular, oblique or diagonal manner through the surface-area portion in which the two packaging-material webs are not sealed to one another.

13. Method according to Claim 11 or 12,
**characterized**
**in that** the packaging-material webs are connected to one another by means of sealing seams or sealing surfaces, in particular using a sealing wax.

14. Method according to one of Claims 11 to 13,
**characterized**
**in that**, in the portion in which the packaging-material webs are not sealed to one another, the two packaging-material webs are provided with at least one structure (7) for tearing the packaging material, wherein these structures (7) are preferably of identical configuration and are arranged congruently in relation to one another.

15. Method according to one of Claims 11 to 14,
**characterized**
**in that** the structure(s) (7) for tearing the packaging material is selected from the group of structures (7) which comprises straight cuts, serrated cuts, undulating cuts, perforations, in particular successive holes or cuts, material cutouts, punched formations, in particular arrow-shaped, triangular and diamond-shaped punched formations, and predetermined breaking points.

## Revendications

1. Emballage de dose individuelle pour des systèmes thérapeutiques transdermiques ou des formes galéniques pelliculaires sous la forme d'un sachet à bord scellé à ouvrir, comprenant deux éléments de matériau d'emballage, qui sont disposés de façon superposée, dont au moins un élément de matériau d'emballage se compose d'un matériau d'emballage résistant à la déchirure, qui présente une faible résistance à la propagation d'une déchirure, dans lequel les deux éléments de matériau d'emballage (11, 12), qui sont assemblés l'un à l'autre en un sac à bord scellé (1) par une soudure continue (3) entourant le produit emballé (5), présentent à une extrémité du sac à bord scellé (1) une patte (2), qui est saillante au-delà du bord scellé (3), dans lequel au moins un des éléments de matériau d'emballage (11, 12) présente une structure (7) destinée à la déchirure du matériau d'emballage dans la région des pattes (2), dans laquelle les pattes (2) ne sont pas soudées l'une à l'autre, et dans lequel la structure (7) destinée à la déchirure du matériau d'emballage est sans contact avec le bord de l'emballage, **caractérisé en ce que** les pattes (2) ont essentiellement la moitié de la largeur du sac à bord scellé (1) et présentent, à leur extrémité qui est éloignée du bord scellé (3), une région de soudage (6) dans laquelle elles sont assemblées l'une à l'autre.

2. Emballage de dose individuelle selon la revendication 1, **caractérisé en ce que** les pattes (2) de deux sacs à bord scellé (1) de mêmes dimensions peuvent se compléter et s'assembler par complémentarité de surface en une région de surface quadrangulaire.

3. Emballage de dose individuelle selon la revendication 1 ou 2, **caractérisé en ce que** la patte (2) est disposée de façon asymétrique et décentrée sur le sac à bord scellé (1).

4. Emballage de dose individuelle selon l'une quelconque des revendications précédentes, **caractérisé en ce que** la résistance à la déchirure du matériau d'emballage se situe entre 20 N et 2000 N, de préférence entre 50 N et 150 N, et de préférence encore entre 70 N et 100 N, mesurée sur les deux éléments de matériau d'emballage (11, 12) assemblés l'un à l'autre, qui forment l'emballage de dose individuelle.

5. Emballage de dose individuelle selon l'une quelconque des revendications précédentes, **caractérisé en ce que** la résistance à la propagation de la déchirure vaut moins de 10 N, de préférence moins de 2 N et de préférence encore moins de 1,5 N, mesurée sur les deux éléments de matériau d'emballage (11, 12) assemblés l'un à l'autre, qui forment l'emballage de dose individuelle.

6. Emballage de dose individuelle selon l'une quelconque des revendications précédentes, **caractérisé en ce que** le rapport de la résistance à la déchirure à la résistance à la propagation de la déchirure se situe dans la plage de 2:1 à 200:1, de préférence dans la plage de 50:1 à 150:1, rapporté à la résistance à la déchirure et à la résistance à la propagation de la déchirure de deux éléments de matériau d'emballage (11, 12) assemblés l'un à l'autre, qui forment l'emballage de dose individuelle.

7. Emballage de dose individuelle selon l'une quelconque des revendications précédentes, **caractérisé en ce que** la force de déchirure pour l'ouverture des deux éléments de matériau d'emballage (11, 12) assemblés l'un à l'autre se situe dans la plage de 1 à 50 N/15 mm de largeur de soudure, de préférence dans la plage de 3 à 20 N/15 mm de largeur de soudure.

8. Emballage selon l'une quelconque des revendications précédentes, **caractérisé en ce que** la structure (7) destinée à la déchirure de l'élément d'emballage est choisie dans le groupe de structures, qui se compose d'entailles droites, d'entailles en dents de scie, d'entailles ondulées, de perforations, en particulier de trous ou d'entailles qui se suivent, d'enlèvements de matière, de découpes, en particulier de découpes en forme de flèches, de triangles et de losanges, et de zones de moindre résistance.

9. Emballage selon l'une quelconque des revendications précédentes, **caractérisé en ce qu'**au moins un des éléments de matériau d'emballage (11, 12), de préférence les deux éléments de matériau d'emballage (11, 12), est/sont formé(s) en une pellicule à une couche ou à plusieurs couches.

10. Emballage selon l'une quelconque des revendications précédentes, **caractérisé en ce que** le produit à emballer (5) emballé dans celui-ci n'est pas accessible à des enfants sans outil et il présente en particulier une sécurité enfants selon la norme DIN EN 14375 et/ou selon ASTM D3475-03a.

11. Procédé de fabrication d'un emballage de dose individuelle selon l'une quelconque de revendications précédentes, comprenant les étapes suivantes:
- procurer une première bande de matériau d'emballage;
- procurer une deuxième bande de matériau d'emballage;
- positionner le produit à emballer (5) sur une des deux bandes de matériau d'emballage;
- superposer et assembler les deux bandes de matériau d'emballage d'une manière telle qu'un compartiment fermé de tous les côtés (4) soit formé pour chaque produit à emballer (5) afin de contenir le produit à emballer (5), au bord ou aux bords duquel les deux éléments de matériau d'emballage (11, 12) sont assemblés l'un à l'autre de façon séparable, dans lequel chaque fois deux emballages de dose individuelle successifs dans la direction de la bande sont disposés par paires, de telle manière que leurs bords scellés tournés l'un vers l'autre soient directement jointifs, et dans lequel, entre les deux emballages de dose individuelle successifs d'une paire, il subsiste une partie de surface, sur laquelle les deux bandes de matériau d'emballage ne sont pas soudées l'une à l'autre;
- appliquer deux structures (7) destinées à la déchirure du matériau d'emballage dans au moins une des deux bandes de matériau d'emballage, dans lequel ces structures (7) sont appliquées sur les longs bords opposés à l'intérieur de la partie de surface, sur laquelle les deux bandes de matériau d'emballage ne sont pas soudées l'une à l'autre; et
- séparer les paires d'emballages successives par une entaille le long d'une ligne, qui est transversale à la direction de bande des bandes de matériau d'emballage, et séparer les emballages de dose individuelle d'une paire d'emballages, du fait que l'on détache les deux emballages de dose individuelle d'une paire d'emballages par une entaille le long d'une ligne qui s'étend sur une première moitié longitudinale des bandes de matériau d'emballage assemblées l'une à l'autre dans la région du bord scellé antérieur d'un emballage de dose individuelle d'une paire d'emballages et sur l'autre moitié longitudinale des bandes de matériau d'emballage assemblées l'une à l'autre dans la région du bord scellé antérieur de l'autre emballage de dose individuelle de la paire d'emballages, de telle manière qu'il se forme des pattes (2) de surface complémentaire.

12. Procédé selon la revendication 11, **caractérisé en ce que** l'entaille destinée à la séparation des deux emballages de dose individuelle formant la paire d'emballages est en forme de S, sigmoïde, angulaire, oblique ou diagonale à travers la partie de surface, dans laquelle les deux bandes de matériau d'emballage ne sont pas soudées l'une à l'autre.

13. Procédé selon la revendication 11 ou 12, **caractérisé en ce que** les bandes de matériau d'emballage sont assemblées l'une à l'autre au moyen de soudures ou de faces soudées, en particulier en utilisant une laque de soudage.

14. Procédé selon l'une quelconque des revendications 11 à 13, **caractérisé en ce que** les deux bandes de matériau d'emballage sont dotées, dans la partie dans laquelle les bandes de matériau d'emballage ne sont pas soudées l'une à l'autre, d'au moins une structure (7) destinée à la déchirure du matériau d'emballage, dans lequel cette/ces structure(s) (7) est/sont de préférence de forme identique et est/sont disposée(s) en recouvrement mutuel.

15. Procédé selon l'une quelconque des revendications 11 à 14, **caractérisé en ce que** la/les structure (s) (7) destinée(s) à la déchirure du matériau d'emballage est/sont choisie(s) dans le groupe de structures (7) qui se compose d'entailles droites, d'entailles en dents de scie, d'entailles ondulées, de perforations, en particulier de trous ou d'entailles qui se suivent, d'enlèvements de matière, de découpes, en particulier de découpes en forme de flèches, de triangles et de losanges, et de zones de moindre résistance.
